(19) **Europäisches Patentamt European Patent Office Office européen des brevets**

(11) **EP 4 775 164 A1**

(12) **EUROPEAN PATENT APPLICATION**
published in accordance with Art. 153(4) EPC

(43) Date of publication:
**15.07.2026 Bulletin 2026/29**

(21) Application number: **23928989.5**

(22) Date of filing: **03.11.2023**

(51) International Patent Classification (IPC):
***A61B 34/10*** (2016.01)  ***G06T 7/30*** (2017.01)

(52) Cooperative Patent Classification (CPC):
**A61B 17/70; A61B 34/10; G06T 7/11; G06T 7/30**

(86) International application number:
**PCT/KR2023/017559**

(87) International publication number:
**WO 2025/053337 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.09.2023 KR 20230119273**

(71) Applicant: **Curexo Inc.**
**Seoul 05814 (KR)**

(72) Inventors:
• **HWANG, Sung Teac**
**Seoul 03622 (KR)**
• **KANG, Hyun Jung**
**Seoul 01614 (KR)**
• **WOO, Dong Gi**
**Seoul 04722 (KR)**

(74) Representative: **Lavoix**
**Bayerstraße 83**
**80335 München (DE)**

(54) **2D IMAGE-BASED AUTOMATIC SURGERY PLANNING METHOD AND SYSTEM**

(57)     Provided is a two-dimensional (2D) image-based surgical planning method and system. The planning method includes: acquiring a first image and a second image from a spinal surgery site; defining a virtual three-dimensional (3D) surgical space and registering the first image and the second image; extracting a first vertebral body region and a first pedicle region from the first image and extracting a second vertebral body region and a second pedicle region within the second vertebral body region from the second image; setting a first screw path passing through a center or a vicinity of the center of the first pedicle region in the first image; and setting, based on the first screw path, an entry point and a target point on a second screw path corresponding to the first screw path in the second pedicle region of the second image.

FIG. 1

## Description

Technical Field

The disclosure relates to an automatic surgical planning method and system in a surgical robot system, and particularly, to a two-dimensional (2D) image-based automatic surgical planning method and system, whereby a surgical planning path may be generated in a three-dimensional (3D) image space using a plurality of 2D images.

Background Art

Spine surgical procedures, such as a pedicle screw fixation surgery using a surgical robot system, require three-dimensional (3D) images, for which an axial view is available. Therefore, rather than a 2D image, a 3D image, for which an axial view is available, for example, a computed tomography (CT) image, is used as an axial view image.

Generally, during intra-operation, a 2D image acquisition device, for example, a so-called C-arm, is used to verify, in real time, whether surgery is proceeding as planned before the surgery. To use the 2D image acquired as described above to navigate surgical tools as planned in advance, the 2D image acquired during intra-operation has to be registered with respect to a 3D image acquired before surgery. However, the process of registering a 2D image to a 3D image may result in delays in surgery time and registration errors due to incomplete registration.

On the other hand, a surgical plan based only on 3D images acquired before surgery without the help of 2D images during surgery can avoid neither the radiation exposure occurring when the 3D images, for example, CT images, are obtained, nor the problem caused by the difference between patient's preoperative conditions and patient's intraoperative condition.

However, if a surgical plan is established based on 2D X-ray images acquired during surgery, it is not necessary to register the images to images acquired before surgery, and the flexibility of changing the surgical plan according to necessity even during surgery may be expected. However, this process requires a surgeon to estimate a location in a 3D space, in which the surgery is to be performed, while viewing the 2D image. As a result, these surgical plans require a surgical experience with a high difficulty level from the surgeon. Even if this is realized, a 2D image lacks image quality and image information according to 3D spatial image information, compared to a 3D CT image, and thus, it is difficult to expect a successful surgery unless the surgery is performed by an experienced surgeon.

Considering these aspects, it is highly desirable to develop a 2D image-based surgical planning method and system which may reduce the problem of radiation exposure, provide performance as good as the performance of a previous 3D image-based surgical planning system, and alleviate the cost burden of a system configuration.

Disclosure

Technical Problem

The disclosure provides a two-dimensional (2D) image-based surgical planning method and system.

The disclosure provides a 2D image-based surgical planning method and system, whereby a three-dimensional (3D) position, posture, and shape of a vertebral body that is a surgical target may be estimated based on a 2D image without a 3D image.

Also, the disclosure provides a method and system, whereby a surgical plan may be established based on a 2D image acquired in real time during surgery without prior imaging and the surgery may be performed based on the established surgical plan.

Technical Solution

A two-dimensional (2D) image-based surgical planning method according to the disclosure includes:

acquiring, via an image acquisition device, a first image in a first direction with respect to a spinal surgery site and a second image in a second direction different from the first direction;

defining, via a spatial registration portion, a virtual three-dimensional (3D) surgical space corresponding to the surgical site and registering, via the spatial registration portion, the first image and the second image to the surgical space;

extracting, via an object separation portion, a first vertebral body region and a first pedicle region corresponding to a vertebra and a pedicle from the first image, and extracting, via the object separation portion, a second vertebral body region and a second pedicle region within the second vertebral body region from the second image;

setting, via a path generating portion, a first screw path passing through a center or a vicinity of the center of the first pedicle region in the first image, the first screw path being perpendicular to an intermediate boundary line passing between the first pedicle region and the first vertebral body region; and

determining, via a coordinate determining portion, a 3D coordinate for insertion of a surgical screw in the surgical space by setting, based on the first screw path, an entry point and a target point on a second screw path corresponding to the first screw path, in the second pedicle region of the second image.

According to one or more embodiments,
a screw entry point and a screw target point may be set on the first screw path, and two normal lines each extending from the entry point or the target point in the first image may pass through the second pedicle region of the second image.

According to one or more embodiments,
the first pedicle region may have an inner edge facing the first vertebral body region and an outer edge at an opposite side to the inner edge, the entry point may be located on or adjacent to the outer edge of the first pedicle region, and the target point may be located within the first vertebral body region.

According to one or more embodiments,
the entry point and the target point in the second vertebral body region may be set on an edge of the second pedicle region located within the second vertebral body region or may be set in the second pedicle region to be adjacent to the edge of the second pedicle region.

According to one or more embodiments,
the entry point may be arranged on an edge of the second pedicle region toward an outer side of the second vertebral body region or arranged in the second pedicle region to be adjacent to the edge of the second pedicle region, and the target point may be arranged on another edge facing the edge of the second pedicle region or arranged in the second pedicle region to be adjacent to the other edge.

According to one or more embodiments,
the method may further include, in the acquiring of the first image in the first direction and the second image in the second direction different from the first direction, calculating an angle of the second direction with respect to the first direction by detecting a posture of an image acquiring portion by using an optical tracking system (OTS).

According to one or more embodiments,
in the registering, the first image and the second image may be registered to the surgical space by reflecting the angle.

A two-dimensional (2D) image-based surgical planning system according to the disclosure includes:

an image acquisition device configured to acquire a first image in a first direction of a spinal surgery site and a second image in a second direction different from the first direction;
a spatial registration portion configured to define a virtual three-dimensional (3D) surgical space corresponding to the surgical site and register the first image and the second image to the surgical space;
an object separation portion configured to extract a first vertebral body region and a first pedicle region corresponding to a vertebra and a pedicle from the first image and extract a second vertebral body region and a second pedicle region within the second vertebral body region from the second image;
a path generating portion configured to set a first screw path passing through a center or a vicinity of the center of the first pedicle region in the first image, the first screw path being perpendicular to an intermediate boundary line passing between the first pedicle region and the first vertebral body region; and
a coordinate determining portion configured to determine a 3D coordinate for insertion of a screw in the surgical space by setting, based on the first screw path, an entry point and a target point of the screw corresponding to the first screw path in the second pedicle region of the second image.

According to one or more embodiments,

the path generating portion may further be configured to
set a screw entry point and a screw target point on the first screw path and allow two normal lines each extending from the entry point or the target point in the first image to pass through the second pedicle region of the second image.

According to one or more embodiments,

the first pedicle region may have an inner edge facing the first vertebral body region and an outer edge at an opposite side to the inner edge, and
the path generating portion may further be configured to
allow the entry point to be located on the outer edge of the first pedicle region or in the first pedicle region to be adjacent to the outer edge and allow the target point to be located within the first vertebral body region.

According to one or more embodiments,

the path generating portion may further be configured to
set the entry point and the target point in the second vertebral body region on an edge of the second pedicle region located within the second vertebral body region or in the second pedicle region to be adjacent to the edge of the second pedicle region.

According to one or more embodiments,

the coordinate determining portion may further be configured to
arrange the entry point on an edge of the second pedicle region toward an outer side of the second vertebral body region or in the second pedicle region to be adjacent to the edge and arrange the target point on another edge facing the edge of the second pedicle region or in the second pedicle region to be adjacent to the other edge.

According to one or more embodiments,
the system may further include an optical tracking system (OTS) configured to calculate an angle of the second direction with respect to the first direction by detecting a posture of the image acquisition device in a process of acquiring the first image in the first direction and the second image in the second direction different from the first direction.
According to one or more embodiments,
the registration portion may further be configured to register the first image and the second image to the surgical space by reflecting the angle.

Advantageous Effects

O-arm or three-dimensional (3D) C-arm devices and portable 3D imaging devices allow 3D viewing of images of a patient, making it possible to establish a surgery plan by looking at areas that cannot be seen in two dimensions. However, the O-arm or 3D C-arm devices and the portable 3D imaging devices cause a lot of radiation exposure to patients and medical staff, take a long imaging time, and are high cost imaging equipment, making it unavailable to be used in many hospitals. On the other hand, two-dimensional (2D) imaging devices are widely used in many hospitals and are inexpensive and easy to use. However, there are downsides to the 2D imaging devices, such as a lack of patient image information due to 2D imaging, increased radiation exposure through multiple imaging, and differences in surgical planning and surgical effectiveness depending on skill level.
A method and system according to the disclosure is a system for automatically generating a surgical plan based only on 2D images without using computed tomography (CT) or a portable 3D imaging device, whereby the difficulty of surgical planning due to 2D image information that lacks information compared to 3D image information may be systematically solved, errors depending on the level of skills as well as radiation exposure may be reduced, and with the automatically generated surgical plan, the overall surgery time may be shortened.

Description of Drawings

FIG. 1 is a flowchart of time-sequential processing operations of a two-dimensional (2D) image-based surgical planning method according to one or more embodiments of the disclosure.
FIG. 2 schematically shows registration of a first image and a second image to a virtual three-dimensional (3D) surgical space and definition of 3D coordinates, according to one or more embodiments of the disclosure.
FIG. 3 shows an output image in which a vertebral body and pedicle are segmented from a lateral-lateral (LL) image by applying a first image segmentation model, according to one or more embodiments of the disclosure.
FIG. 4 shows an output image in which a vertebral body and pedicle are segmented from an anterior-posterior (AP) image by applying a second image segmentation model, according to one or more embodiments of the disclosure.
FIG. 5 schematically shows a generation process of a deep learning model applied by one or more embodiments of the disclosure.
FIG. 6 shows an image in which a vertebral body and pedicle are labeled in a plurality of first images for learning, according to one or more embodiments of the disclosure.
FIG. 7 shows an image in which a vertebral body and pedicle are labeled in a plurality of second images for learning, according to one or more embodiments of the disclosure.
FIG. 8 shows results of labeling segmented targets in a first image and a second image, according to one or more embodiments of the disclosure.

FIG. 9 illustrates a first selected target L1, according to one or more embodiments of the disclosure.

FIG. 10 illustrates a vertebral body region (A) and a pedicle region (B) set in a first image, according to one or more embodiments of the disclosure.

FIG. 11 is a y-z planar graph showing vertex coordinates of a vertebral body region (A) and a pedicle region (B) set in a first image and a midline between these regions, according to one or more embodiments of the disclosure.

FIG. 12 is a y-z planar graph showing a temporary straight line obtained by rotating the midline of FIG. 11 by 90 degrees, according to one or more embodiments of the disclosure.

FIG. 13 is a y-z planar graph showing a first screw path D aligned in parallel with a temporary straight line D', according to one or more embodiments of the disclosure.

FIG. 14 is a y-z planar graph showing a set coordinate of a target point T or a target point of a surgical screw on a screw path, according to one or more embodiments of the disclosure.

FIG. 15 is an image showing a state in which a second vertebral body region (a) and second pedicle regions (b) and (b') are extracted or separated through a first image segmentation model, according to one or more embodiments of the disclosure.

FIG. 16 is an x-z planar graph showing, by dotted lines, oval-shaped second pedicle regions (b) and (b') within a second vertebral body region (a) with vertices (a1), (a2), (a3), and (a4), according to one or more embodiments of the disclosure.

FIG. 17 shows a schematic structure of a surgical robot system according to one or more embodiments of the disclosure.

Mode for Invention

Hereinafter, preferred embodiments of the present inventive concept will be described in detail with reference to the accompanying drawings. However, the embodiments of the present inventive concept may be modified into various other forms, and the scope of the present inventive concept should not be construed as being limited to the embodiments described in detail below. It is preferable that the embodiments of the present inventive concept be interpreted as being provided to more completely explain the present inventive concept to those with average knowledge in the art. Identical symbols refer to identical elements throughout. Furthermore, various elements and areas in the drawings are schematically drawn. Thus, the inventive concept is not limited by the relative sizes or distances shown in the accompanying drawings.

The terms, such as "first," "second," etc., may be used to describe various components, but the components shall not be limited by the terms. These terms are used only for distinguishing one element from another element. For example, without deviating from the scope of the claims of the disclosure, a first element may be referred to as a second element, and in contrast, the second element may be referred to as the first element.

Terms used in the present application are used only for describing particular embodiments of the disclosure and are not intended to limit the disclosure. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless defined otherwise, all of the terms used herein, including technical terms and scientific terms, have the same meaning as the meaning commonly understood by one of ordinary skill in the art. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

When a certain embodiment may be implemented differently, a specific process order may be performed differently from the described order. For example, two consecutively described processes may be performed substantially at the same time or performed in an order opposite to the described order.

Hereinafter, a two-dimensional (2D) image-based surgical planning method and system will be described in detail according to one or more embodiments.

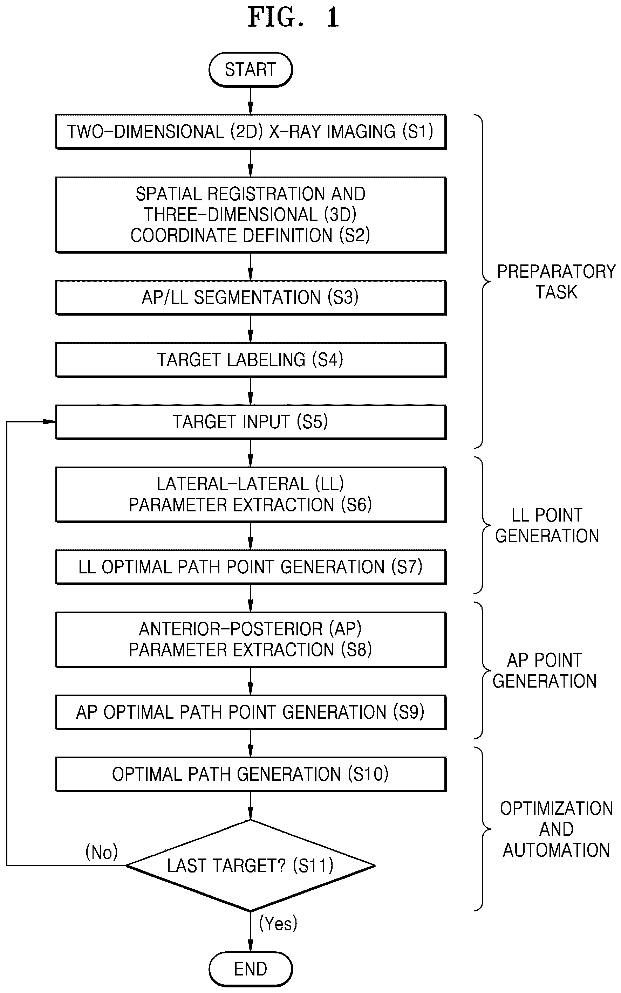

FIG. 1 is a time-sequential flowchart of a 2D image-based surgical plan according to the disclosure.

The process in FIG. 1 includes operations S5 to S10 that are repeatedly performed on a plurality of vertebral bodies which are targets, and when there is only one vertebral body, a surgical plan for one vertebra is established by performing the process once.

FIG. 1 is described as below according to each operation.

Overall, the method according to the disclosure includes a preparatory work process, an LL point generation process, an AP point generation process, and an optimization and automation process.

<Operation S1>

Operation S1 is an operation in which a 2D image with respect to a patient's affected area is obtained in two directions as a first image and a second image. A lateral-lateral (LL) image may be acquired, for example, as the first image, and an anterior-posterior (AP) image is acquired, for example, as the second image. The two directions in which the first and second images are acquired may be perpendicular to each other, may intersect each other, or may intersect each other while maintaining an arbitrary angle between each other to be almost perpendicular to each other. For the acquisition of the first and second images, an X-ray source in the form of a point source may be implemented at one side of the patient's affected area, namely, a vertebral area which is a surgical target, and an image acquisition device having an X-ray detector, for example, a so-called C-arm device, may be implemented at the opposite side. According to another embodiment of the disclosure, a marker attached on a patient's body portion and markers of calibrators for estimating the positions of a source and a detector, and a commercialized optical tracking system (OTS) having a sensor for detecting three-dimensional (3D) positions of the markers may be used to measure the angle between the first direction and the second direction in which the first image and the second image are acquired.

<Operation S2>

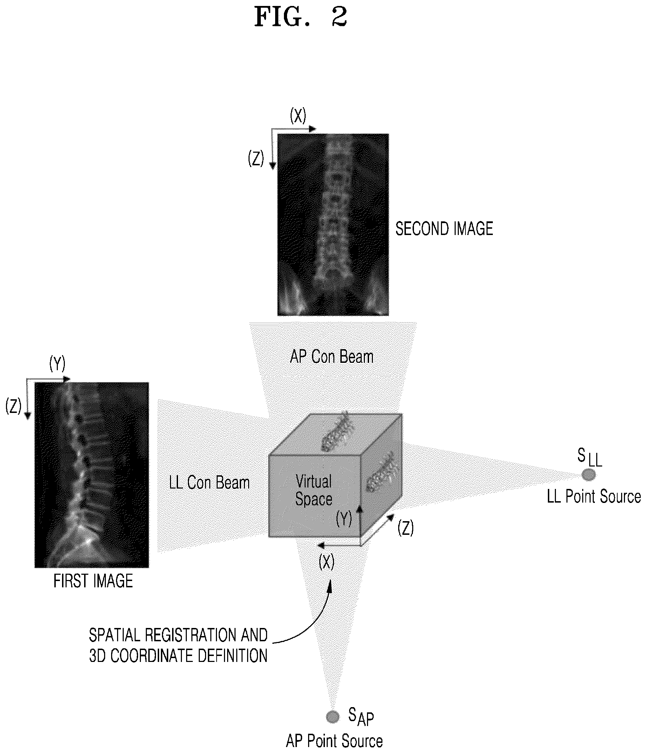

In operation S2, the first image and the second image are registered to a virtual 3D surgical space, and coordinates with respect to pixels of the first and second images in a 3D space may be determined or defined. FIG. 2 schematically shows registration of a first image and a second image acquired by a cone-shaped or conical beam-shaped X-ray to a virtual 3D surgical space and definition of 3D coordinates. As shown, a source and a detector geometrically have a perspective projection relationship due to the cone-shaped X-ray beam.
The virtual surgical space, that is, a virtual space, has axes in X, Y, and Z directions. In an AP image, a top and bottom direction (head-foot) indicates the Z axis and a left and right direction (left arm-right arm) indicates the X axis, and in an LL image, the left and right direction (belly-back) indicates the Y axis and the top and bottom direction (head-foot) likewise indicates the Z axis. Thus, the first image, for example, the LL image, is parallel with a Y-Z plane, and the second image, for example, the AP plane, is parallel with the an X-Z plane.
According to the present embodiment, first images of a surgical site, for example, the AP image and the LL image, are acquired through a 2D image acquisition device such as C-arm equipment, etc. and the image acquisition device such as the C-arm equipment, etc. is registered to a coordinate system with respect to a marker placed at a patient's body portion or other markers of calibrators established in a surgical space. The Patent Registration KR 2203544 of the present applicant discloses a technique for registering a 2D image to a 3D space, and the present application fully incorporates No. 544' by reference. However, the disclosure is not technically limited to specific registration techniques.

<Operation S3>

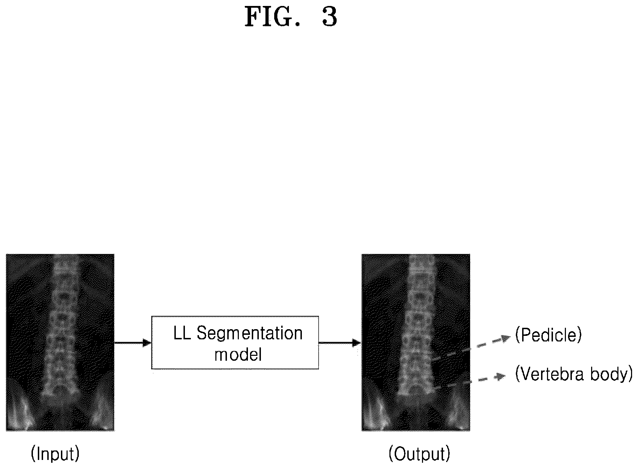

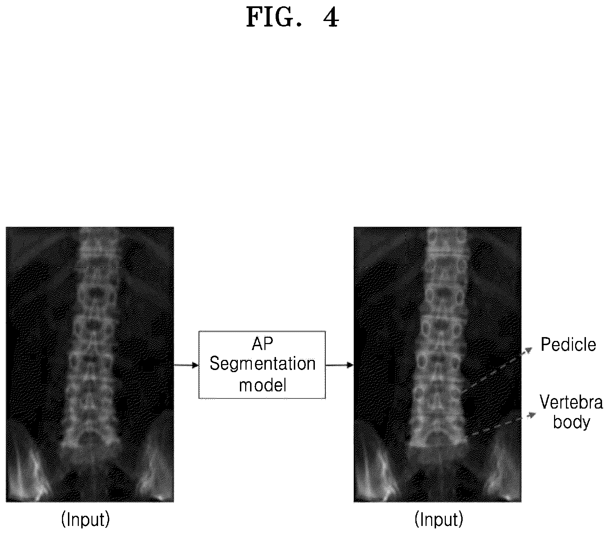

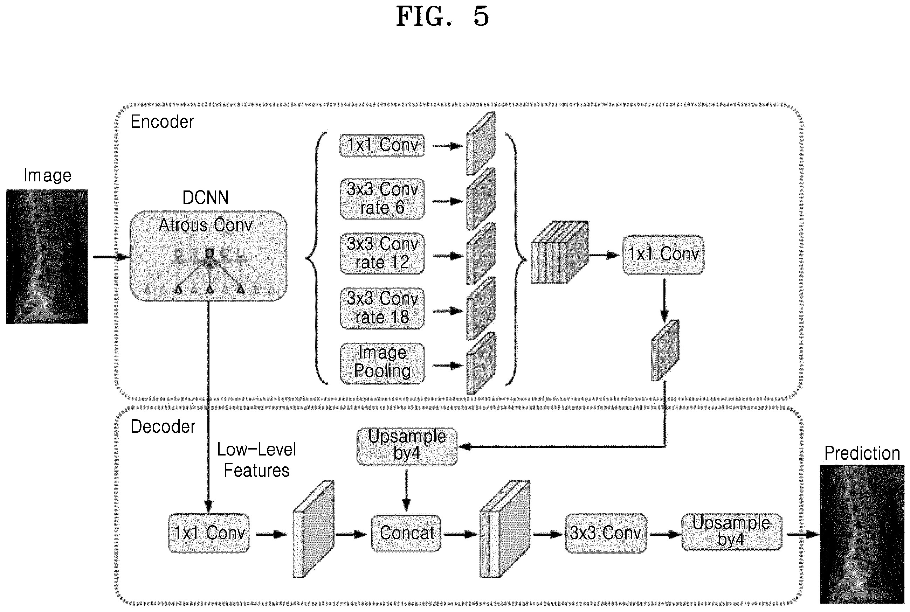

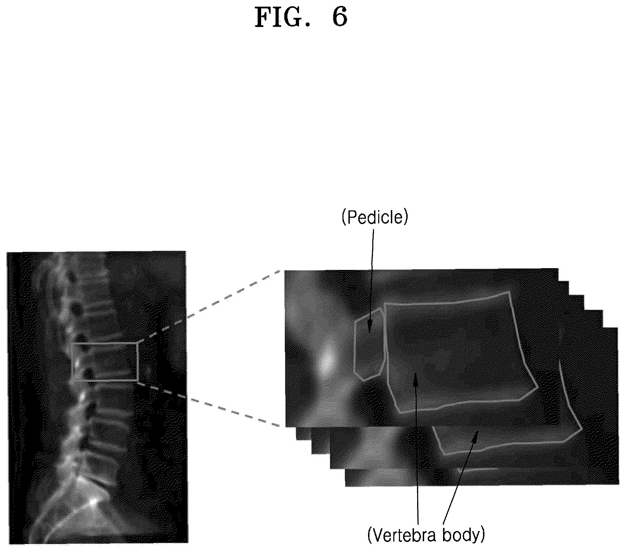

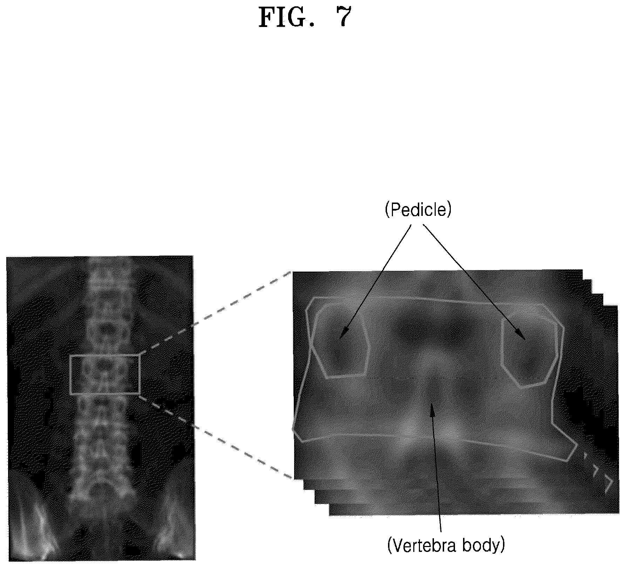

In operation S3, the extraction or segmentation of a vertebral body and pedicle as target objects from the first and second images obtained in the process described above and registered to the virtual 3D surgical space, is performed.
The segmentation of the object may be performed by applying an LL segmentation model and an AP segmentation model trained by deep learning. FIG. 3 shows an output image in which the vertebral body and the pedicle are segmented from an LL image by applying a first image segmentation model, and FIG. 4 shows an output image in which the vertebral body and the pedicle are segmented from an AP image by applying a second image segmentation model.
The segmentation models as described above may be based on the DeepLabv3+ model as shown in FIG. 5. This model is one of the deep learning models used in the field of computer vision and is used to perform extraction or segmentation tasks. This model is based on a deep convolutional neural network (CNN) architecture and particularly, has an encoder and decoder structure so as to be suitable for image segmentation.
Training of the model includes the same process as general deep learning training. For the training of the model as described above, various vertebra-related image resources collected for training are required. Training images include a first image for training captured in a first direction and a second image for training captured in a second direction, and labeling at the pixel level is performed on the first image for training and the second image for training and then a learning process is performed, as illustrated in FIGS. 4 and 5, so that the targeted first and second image segmentation models may be acquired. FIG. 6 described above shows the vertebral body and the pedicle labelled in a plurality of first images for training and FIG. 7 described above shows the vertebral body and the pedicle labelled in a plurality of second images for training.

<Operation S4>

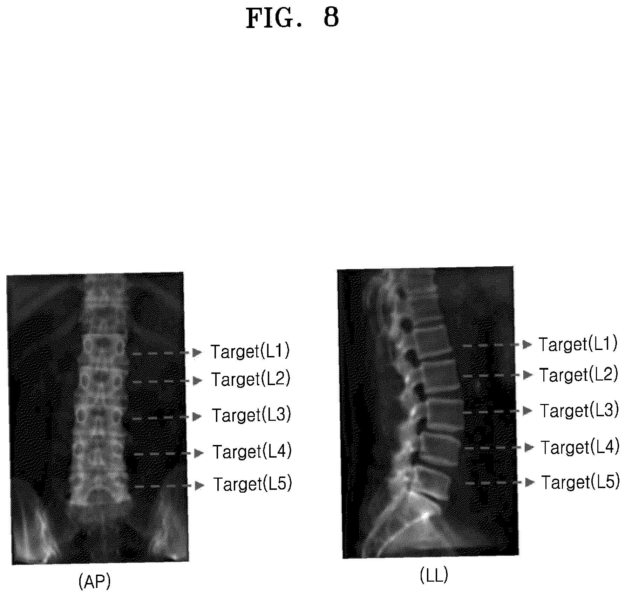

In this operation, labeling is performed on targets segmented in a previous operation. FIG. 8 shows results of labeling the segmented targets in an AP image and an LL image.

As shown in FIG. 8, the segmented targets are labeled as L1, L2, L3, L3, L4, and L5 sequentially from the top, and the same label is assigned to the same vertebral body.

<Operation S5>

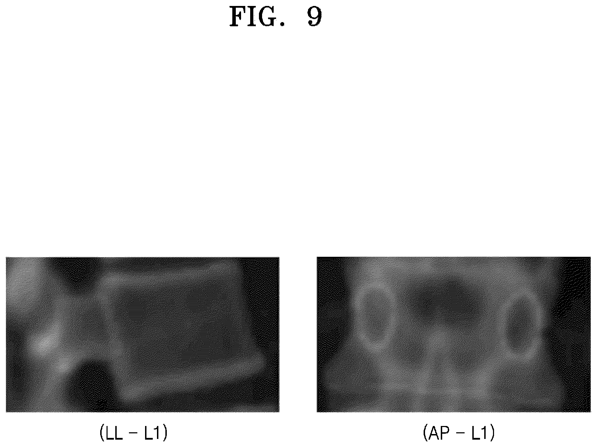

In this operation, before a process of extracting an LL point, one target is selected from among a plurality of targets. For example, as shown in FIG. 9, the target L1 may be selected first and may undergo a subsequent process.

<Operation S6>

A first screw path may be determined on an LL image plane by performing extraction of an LL parameter with respect to the labelled selected target. When planning the path, the path has to be set not to deviate from pedicle and vertebral body regions. Here, if the slope of only one of the vertebral body region or the pedicle region is reflected, the path may deviate from the remaining region, and in order to prevent path planning failure due to detection errors occurring during object segmentation, the screw path may be set as the average slope of the slopes of the two regions.

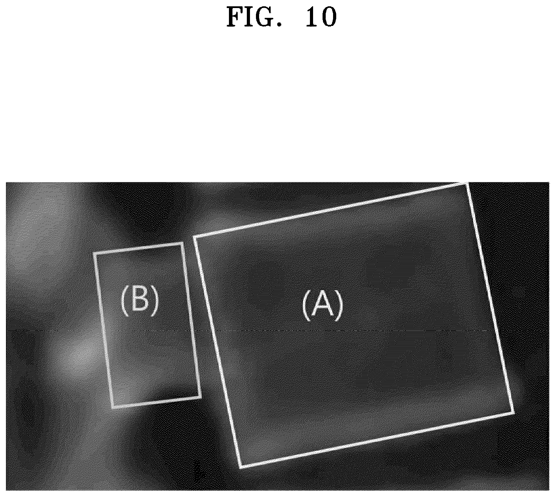

To this end, first, the region of interest (ROI) with respect to the pedicle and vertebral body is set. In FIG. 10, (A) is the vertebral body region, and (B) is the pedicle region. The ROI can be obtained by the first image segmentation model described above.

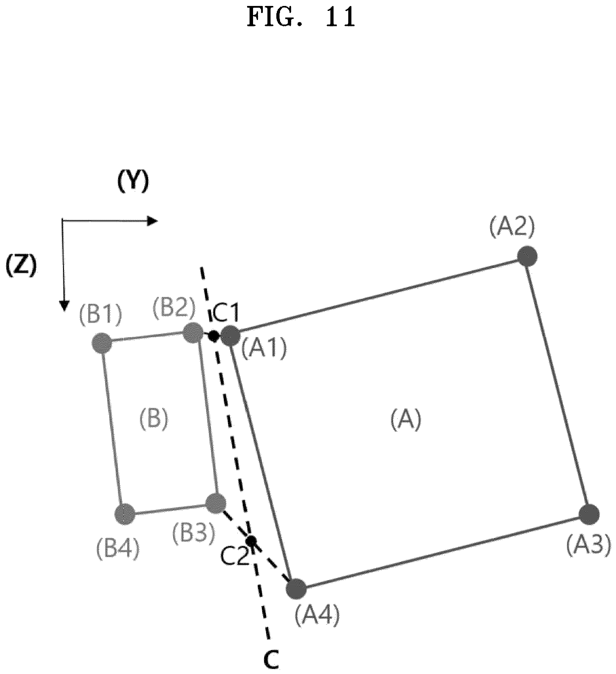

As illustrated in FIG. 11, the vertebral body region (A) is a quadrangular region with four vertices (A1), (A2), (A3) and (A4), the pedicle region (B) is a quadrangular region with four vertices (B1), (B2), (B3) and (B4), and the vertebral body region (A) and the pedicle region (B) are adjacent to each other. A midline C passes between adjacent sides of the two adjacent regions.

The middle line C passes through the exact center between two adjacent sides (B2)-(B3) and (A1)-(A4) of the both regions (A) and (B), and intersection points C1 and C2 are located on the middle line C. The intersection point C1 is located in the middle of a line segment connecting the vertices (B2) and (A1) of the both regions, and the intersection point C2 is located in the middle of a line segment connecting the vertices (B3) and (A4). Therefore, a y coordinate (C1.y) of C1 is (A1.y+B2.y)/2, and a z coordinate (C1.z) of C1 is (A1.z+B2.z) / 2. Also, a y coordinate (C2.y) of C2 is (A4.y+B3.y) / 2, and a z coordinate (C2.z) of C2 is (A4.z+B3.z) / 2.

Thus, the midline C has a planar coordinate of an LL image, that is, an average slope Ca of the slopes of both regions on a y-z plane. The midline C serves as a reference for setting the first screw path on an LL image plane, and the coordinates C1(y, z) and C2(y, z) of the intersection points C1 and C2 in the y-z planar coordinate system and a slope Ca of the midline C are expressed by the equations below.

$$C1(y, z) = \left(\frac{A1.y+B2.y}{2}, \frac{A1.z+B2.z}{2}\right)$$

$$C2(y, z) = \left(\frac{A4.y+B3.y}{2}, \frac{A4.z+B3.z}{2}\right)$$

$$Ca = \frac{C2.z - C1.z}{C2.y - C1.y}$$

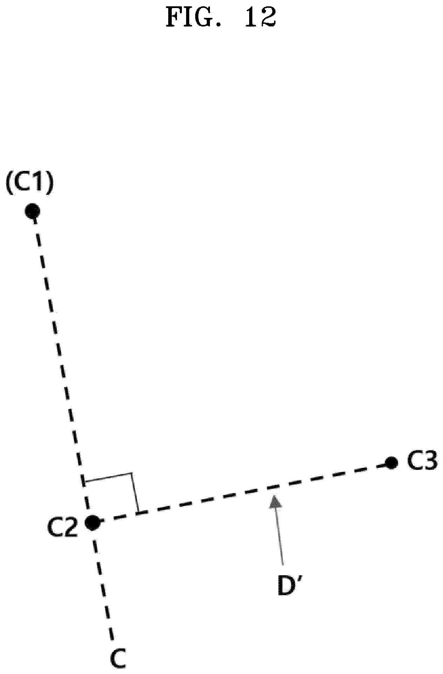

FIG. 12 is a graph showing a temporary point C3 in the middle of a process of setting the first screw path from the midline C between the pedicle region (B) and the vertebral body region (A) on the y-z plane.

The coordinate of the temporary point C3 corresponds to the coordinate obtained by rotating the intersection point C1 by 90 degrees with respect to the intersection point C2 on the midline C. That is, when the coordinate of C1 on the y-z planar coordinate system is (y1, z1), the coordinate of C3 becomes (z1, -y1). As a result, a temporary straight line D' connecting the intersection point C2 with the temporary point C3 is a normal line perpendicular to the midline C, and the straight line equation of the normal line is D':Z = Da(Y - C2.y) + C2.z.

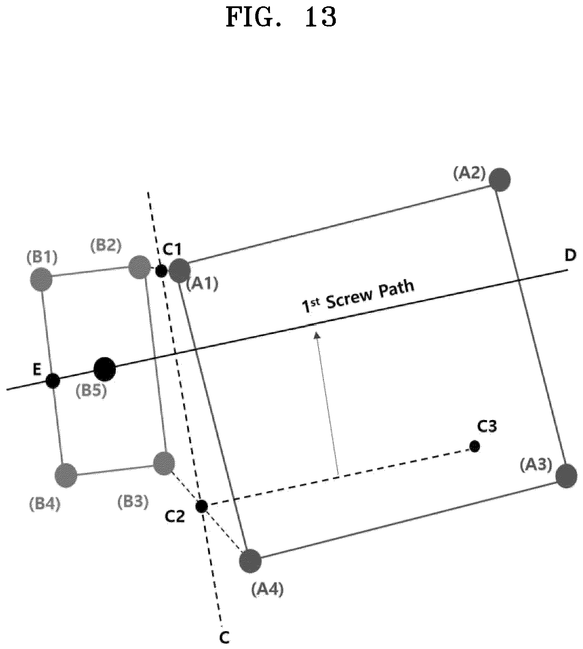

FIG. 13 shows a first screw arrangement line, that is, a first screw path D, arranged in parallel with the temporary straight line D'. The first screw path D is perpendicular to the midline C and parallel with the temporary straight line D'. The temporary straight line D' is shifted in parallel by a certain distance so as to pass through a center (B5) or the near center of the pedicle region (B) having the four vertices such as (B1), (B2), (B3), and (B4), in order to determine the first screw path D on the y-z plane.

A y coordinate (B5.y) and a z coordinate (B5.z) of (B5) are expressed as follows.

$$B5.y = (B1.y + B2.y + B3.y + B4.y) / 4$$

$$B5.z = (B1.z + B2.z + B3.z + B4.z) / 4$$

The straight line equation of the first screw path D having a slope Da is expressed as follows.

$$D:Z = Da (Y - C2.y) + C2.z$$

$$Da = (C3.z - C2.z) / (C3.y - C2.y)$$

The first screw path D expressed by the straight line equation D:z as described above generates an intersection point E as the first screw path D passes through outer sides (BS) and (B1)-(B4) of the pedicle, and the intersection point E becomes an entry point of a surgical screw which is to be described below.
The outer side BS of the pedicle is expressed by the equation as below.

$$BS: Z = Ba(Y - B1.y) + B1.z$$

$$Ba = (B4.z - B1.z) / (B4.y - B1.y)$$

<Operation S7>

In this operation, the coordinates of an entry point of a screw located on the first screw path D and a target point or a terminating point where the tip of the surgical screw reaches are calculated.
With respect to coordinates (E.y, E.z) of the entry point E located on the outer side of the pedicle, it is possible to calculate the y coordinate (E.y) and the z coordinate (E.z) of the entry point by using the straight line equation D:Z of the first screw path and a linear simultaneous equation applying the straight line equation BS:Z of the outer side (BS) of the pedicle.

$$D:Z = Da (Y - C2.y) + C2.z$$

$$BS: Z = Ba(Y - B1.y) + B1.z$$

First, Y and Z of the two same straight lines are the same at (E.y) and (E.z), and thus, the two lines are calculated as below.

$$Da (Y - C2.y) + C2.z = Ba(Y - B1.y) + B1.z$$

The value of (E.y) may be obtained by gathering the sections with respect to Y in each side and separating Y from each other in the equation above.

$$E.y = Y = (B1.z - C2.z + Ba * B1.y - Da * C2.y) / (Da - Ba)$$

By substituting (E.y) obtained in the equation above into the straight line equation D:Z of the screw path, the coordinate (E.z) of the entry point in the z direction may be obtained.

$$E.z = Z = Da (E.y - C2.y) + C2.z$$

The calculation process above is one of various calculation methods to obtain the coordinates of the entry point E, which may be calculated by other methods, and it is obvious that a specific calculation method does not limit the technical scope of the disclosure.

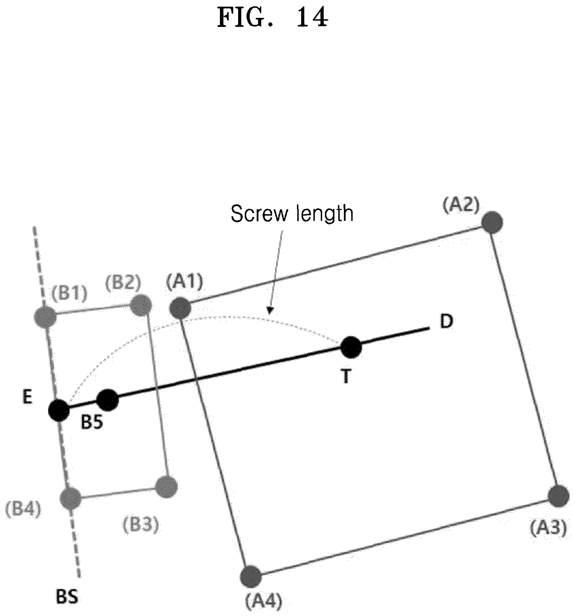

FIG. 14 shows a set coordinate of a target point T or a terminating point of a surgical screw on the screw path on the y-z plane. The coordinate of the target point or the terminating point is determined by the total length Ls of the screw to be used. According to an embodiment according to the disclosure, the total length Ls of the screw is set not to exceed the length of the side (A1)-(A2) of the vertebral body region in a direction in which the surgical screw advances, and the diameter Ds of the screw is set not to be greater than the length of the outer side (B1)-(B4) where the entry point of the screw is located.

$$Ls = \sqrt{(A1.y - A2.y)^2 + (A1.z - A2.z)^2}$$

$$Ds = \sqrt{(B1.y - B4.y)^2 + (B1.z - B4.z)^2}$$

Here, coordinates T(y, z) of the target point are expressed by the equations below.

$$T.y = E.y + Ls * Cos(arctan(Da))$$

$$T.z = E.z + Ls * Sin(arctan(Da))$$

<Operation S8>

In this operation, a parameter is extracted for determining a second screw path on a second image plane, that is, an x-z plane of an AP image, from the screw path obtained on the y-z plane.

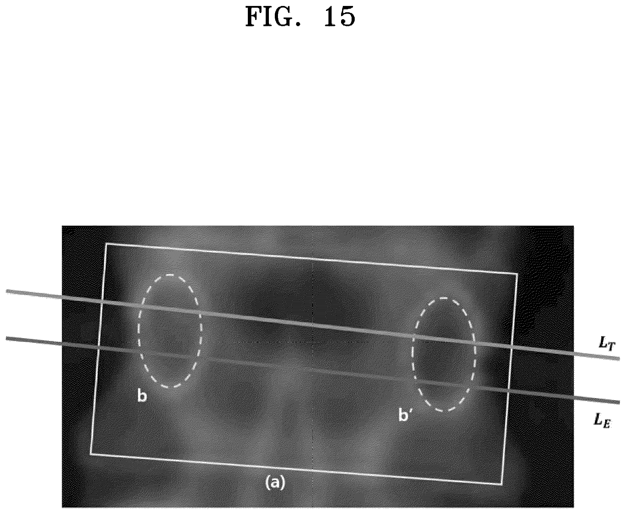

FIG. 15 shows a state in which a second vertebral body region (a) and left and right second pedicle regions (b) and (b') within the second vertebral body region (a) are extracted or separated through an AP segmentation model.
In FIG. 15, two straight lines $L_T$ and $L_E$ inclined to the right traverse the second vertebral body region (a), while passing through the left and right second pedicle regions (b) and (b') within the vertebral body region (a).
Of the two straight lines above, $L_T$ is a target point setting line, and $L_E$ is an entry point setting line. As described above, since the AP plane of the second image is registered to the LL plane of the first image, the entry point and the target point of the LL plane of the first image may be formed on the target point setting line $L_T$ and the entry point setting line $L_E$ on the AP plane of the second image.
According to an embodiment of the disclosure, the entry point setting line $L_E$ is a normal line extending from the entry point on the y-z plane in an x direction perpendicular to the y-z plane, and the target point setting line $L_T$ is a normal line extending from the target point on the y-z plane in the x direction perpendicular to the y-z plane.

<Operation S9>

In this operation, the entry point and the target point on the x-z plane are formed on the entry point setting line and the target point setting line.
The entry point and the target point on the x-z plane determine or form the second screw path on the x-z plane and are formed on an edge line of the pedicle. This is to set the entry path of the screw to be safest as possible. The entry point and the target point are set such that the second screw path for screws at both sides is toward a central direction of the vertebral body from an outer direction of the vertebral body.

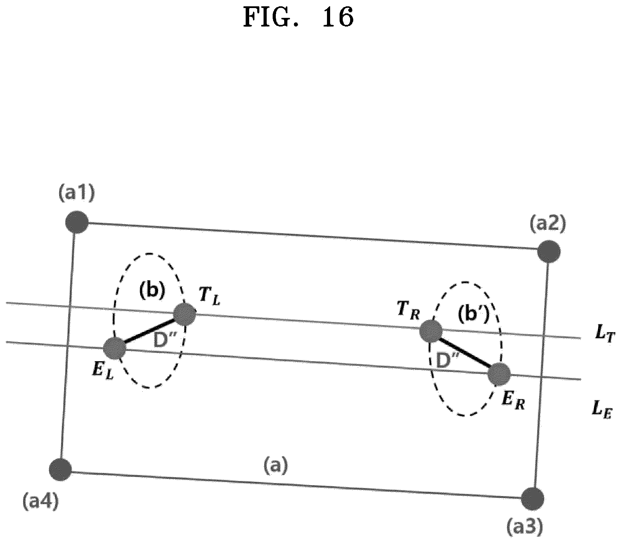

FIG. 16 shows, on the x-z plane, the oval-shaped second pedicle regions (b) and (b') within the second vertebral body region (a) with the vertices (a1), (a2), (a3), and (a4).
As shown in FIG. 16, the target point setting line $L_T$ and the entry point setting line $L_E$ described above form an intersection point on an edge line of the second pedicle regions (b) and (b') while passing through the both second pedicle regions (b) and (b'). There are 4 intersection points in total. 2 intersection points are formed by the target point setting line $L_T$ and 2 intersection points are formed by the entry point setting line $L_E$.
Among four intersection points by the entry point setting line $L_E$ from among the intersection points, two intersection points located outside the second vertebral body region (a) are selected as screw entry points $E_L$ and $E_R$, and among four intersection points by the target point setting line $L_T$ from among the intersection points, two intersection points located at a central area of the second vertebral body region (a) are selected as screw target points $T_L$ and $T_R$. Thus, left and right straight lines D" connecting the left entry point $E_L$ with the left target point $T_L$ in the pedicle region (b) and connecting the right entry point $E_R$ with the right target point $T_R$ in the pedicle region (b'), respectively, are the second screw path.

<Operation S10>

In this operation, through the coordinates of the screw insertion point and target point on the y-z plane and the coordinates of the screw insertion point and target point on the x-z plane, obtained by the same method as described above, 3D coordinates in a surgical space are obtained.

In this operation, the coordinates $E_L(x, z)$, $E_R(x, z)$, $T_L(x, z)$ and $T_R(x, z)$ of the screw entry points $E_L$ and $E_R$ and target points $T_L$ and $T_R$ on the x-z plane, and the coordinate (E.y) of the entry point and the coordinate (E.z) of the target point on the y-z plane may be used to obtain the coordinates $E(x,y,z)$ of the screw entry point and the coordinates $T(x,y,z)$ of the target point in the 3D surgical space. Such acquisition of the coordinates in the 3D surgical space is possible, because the first image and the second image are registered to a virtual 3D surgical space, as described above.

The processes described above are performed with respect to one target. However, when there are multiple targets, that is, when there are a plurality of vertebral bodies, which are the surgical objects, and when the last target has not yet been reached in operation S11, the processes may return to operation S5 and operation S5 to operation S10 are repeatedly performed before the processes are ended.

According to the embodiment according to the disclosure as described above, the screw path in the 3D surgical space may be successfully and automatically planed by using only the 2D images during surgery. The method and system according to the embodiment according to the disclosure uses a 2D imaging device, and thus, may be implemented at low costs. The method and system according to the disclosure is a system for automatically generating a surgical plan based only on 2D images without using computed tomography (CT) or a portable 3D imaging device, whereby the difficulty of surgical planning due to 2D image information lacking information compared to 3D image information may be systematically solved, errors depending on the difference in the level of skills and the radiation exposure may be reduced, and by automatically generating the surgical plan, the overall surgery time may be shortened.

An embodiment according to the disclosure may be implemented by a computer program medium storing software for performing an image registration method on a computer. Additionally, the disclosure may be implemented by an image registration device which may include a processor, a memory, a display, etc. performing the image registration method described above.

Additionally, the disclosure may also be implemented by a surgical robot system based on the image registration method described above.

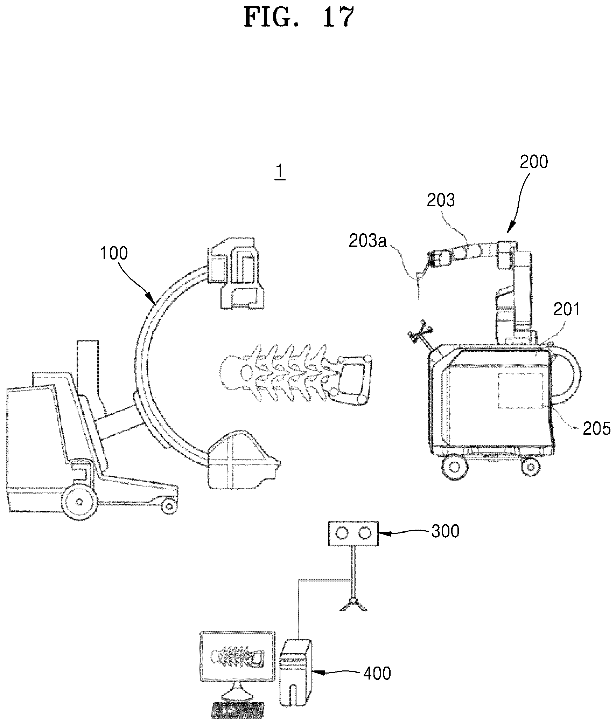

Referring to FIG. 17, a surgical robot system 1 according to one or more embodiments of the disclosure includes a 2D image acquisition device 100 as a main image acquisition device. In addition, the robot system (1) is formed by including a surgical robot 200, a position sensor 300, and a navigation system 400, and the surgical robot 200 is formed by including a main body 201, a robot arm including an end effector 203a, and a robot controller 205.

According to an embodiment according to the disclosure, except for the image acquisition device described above, a spatial registration portion, an object separation portion, a path generating portion, and a coordinate determining portion may be functionally included in the devices described above, and the spatial registration portion, etc. may be included in the navigation system 400. Also, the object separation portion, the path generating portion, the coordinate determining portion, etc. may be implemented by surgical planning software. In particular, the object separation portion may perform extraction or separation of a vertebral body and pedicle from a first image and a second image in 2D by applying an LL segmentation model and an AP segmentation model trained by deep learning.

The 2D image may be obtained by the 2D image acquisition device, for example, a so-called C-arm device having an X-ray source and a detector that are placed on both sides thereof and face each other with a patient undergoing surgery therebetween. That is, according to an embodiment according to the disclosure, a C-arm imaging device may be used as the 2D image acquisition device 100.

The 2D image acquisition device obtains a 2D first image, for example, an LL image, and a 2D second image, for example, an AP image, with respect to a patient's surgical site, in order to obtain a 3D screw path during surgery. The robot arm 203 is fixedly mounted on the robot body 201, and includes, on an end thereof, the end effector 203a, from which the surgical tool can be attached or detached. The position sensor 300 is implemented by an OTS that tracks a real-time position of the surgical tool or the end effector 203a through marker recognition. The controller 205 is provided in the robot body 201 and controls the robot arm 203 according to a surgical plan determined during surgery according to the disclosure and control software. The navigation system 400 performs the image registration method described above to display, through a display, information about a surgical tool or a surgical plan about an implant on the 2D image acquired during surgery or display a real time position of the surgical tool or the implant on the 2D image or in some cases, a 3D image acquired before surgery, thereby assisting the doctor in performing the surgery. To this end, the display that allows the doctor to view, with the naked eye, during surgery, the real time position of the surgical tool, etc. in comparison with the surgical plan and the surgical status, may be connected to the navigation system 400.

Although various embodiments of the disclosure have been described in detail above, one of ordinary skill in the art may implement the disclosure by modifying the embodiments of the disclosure in various ways without deviating from the concept and the range of the disclosure defined in the accompanying claims. Therefore, changes in the embodiments of the disclosure shall not deviate from the description of the disclosure.

**Claims**

1. A two-dimensional (2D) image-based surgical planning method comprising:

   acquiring a first image in a first direction with respect to a spinal surgery site and a second image in a second direction different from the first direction;

   defining a virtual three-dimensional (3D) surgical space corresponding to the surgical site and registering the first image and the second image to the surgical space;

   extracting a first vertebral body region and a first pedicle region corresponding to a vertebra and a pedicle from the first image, and extracting a second vertebral body region and a second pedicle region within the second vertebral body region from the second image;

   setting a first screw path passing through a center or a vicinity of the center of the first pedicle region in the first image, the first screw path being perpendicular to an intermediate boundary line passing between the first pedicle region and the first vertebral body region; and

   determining a 3D coordinate for insertion of a surgical screw in the surgical space by setting, based on the first screw path, an entry point and a target point or terminating point on a second screw path corresponding to the first screw path, in the second pedicle region of the second image.

2. The method of claim 1, wherein a screw entry point and a screw target point are set on the first screw path, and two normal lines each extending from the entry point or the target point in the first image pass through the second pedicle region of the second image.

3. The method of claim 1, wherein the first pedicle region has an inner edge facing the first vertebral body region and an outer edge at an opposite side to the inner edge, the entry point is located on or adjacent to the outer edge of the first pedicle region, and the target point is located within the first vertebral body region.

4. The method of claim 3, wherein the entry point and the target point in the second vertebral body region are set on an edge of the second pedicle region located within the second vertebral body region or set in the second pedicle region to be adjacent to the edge of the second pedicle region.

5. The method of claim 3, wherein the entry point is arranged on an edge of the second pedicle region toward an outer side of the second vertebral body region or arranged in the second pedicle region to be adjacent to the edge of the second pedicle region, and the target point is arranged on another edge facing the edge of the second pedicle region or arranged in the second pedicle region to be adjacent to the other edge.

6. The method of any one of claims 1 to 5, further comprising in the acquiring of the first image in the first direction and the second image in the second direction different from the first direction, calculating an angle of the second direction with respect to the first direction by detecting a posture of an image acquiring portion by using an optical tracking system (OTS).

7. The method of claim 6, wherein, in the registering, the first image and the second image are registered to the surgical space by reflecting the angle.

8. A two-dimensional (2D) image-based surgical planning system comprising:

   an image acquisition device configured to acquire a first image in a first direction of a spinal surgery site and a second image in a second direction different from the first direction;

   a spatial registration portion configured to define a virtual three-dimensional (3D) surgical space corresponding to the surgical site and register the first image and the second image to the surgical space;

   an object separation portion configured to extract a first vertebral body region and a first pedicle region corresponding to a vertebra and a pedicle from the first image and extract a second vertebral body region and a second pedicle region within the second vertebral body region from the second image;

   a path generating portion configured to set a first screw path passing through a center or a vicinity of the center of the first pedicle region in the first image, the first screw path being perpendicular to an intermediate boundary line passing between the first pedicle region and the first vertebral body region; and

a coordinate determining portion configured to determine a 3D coordinate for insertion of a screw in the surgical space by setting, based on the first screw path, an entry point and a target point of the screw corresponding to the first screw path in the second pedicle region of the second image.

9. The system of claim 8, wherein the path generating portion is further configured to set a screw entry point and a screw target point on the first screw path and allow two normal lines each extending from the entry point or the target point in the first image to pass through the second pedicle region of the second image.

10. The system of claim 8, wherein the first pedicle region has an inner edge facing the first vertebral body region and an outer edge at an opposite side to the inner edge, and
the path generating portion is further configured to allow the entry point to be located on the outer edge of the first pedicle region or in the first pedicle region to be adjacent to the outer edge and allow the target point to be located within the first vertebral body region.

11. The system of claim 10, wherein the path generating portion is further configured to set the entry point and the target point in the second vertebral body region on an edge of the second pedicle region located within the second vertebral body region or in the second pedicle region to be adjacent to the edge of the second pedicle region.

12. The system of any one of claims 8 to 11, wherein the coordinate determining portion is further configured to arrange the entry point on an edge of the second pedicle region toward an outer side of the second vertebral body region or in the second pedicle region to be adjacent to the edge and arrange the target point on another edge facing the edge of the second pedicle region or in the second pedicle region to be adjacent to the other edge.

13. The system of claim 12, further comprising an optical tracking system (OTS) configured to calculate an angle of the second direction with respect to the first direction by detecting a posture of the image acquisition device in a process of acquiring the first image in the first direction and the second image in the second direction different from the first direction.

14. The system of claim 13, wherein the registration portion is further configured to register the first image and the second image to the surgical space by reflecting the angle.

15. The system of any one of claims 8 to 11, further comprising an optical tracking system (OTS) configured to calculate an angle of the second direction with respect to the first direction by detecting a posture of the image acquisition device in a process of acquiring the first image in the first direction and the second image in the second direction different from the first direction.

16. The system of claim 13, wherein the registration portion is further configured to register the first image and the second image to the surgical space by reflecting the angle.

**Amended claims under Art. 19.1 PCT**

1. A two-dimensional (2D) image-based surgical planning method comprising:

   acquiring a first image in a first direction with respect to a spinal surgery site and a second image in a second direction different from the first direction;
   defining a virtual three-dimensional (3D) surgical space corresponding to the surgical site and registering the first image and the second image to the surgical space;
   extracting a first vertebral body region and a first pedicle region corresponding to a vertebra and a pedicle from the first image, and extracting a second vertebral body region and a second pedicle region within the second vertebral body region from the second image;
   setting a first screw path passing through a center or a vicinity of the center of the first pedicle region in the first image, the first screw path being perpendicular to an intermediate boundary line passing between the first pedicle region and the first vertebral body region; and
   determining a 3D coordinate for insertion of a surgical screw in the surgical space by setting, based on the first screw path, an entry point and a target point or terminating point on a second screw path corresponding to the first screw path, in the second pedicle region of the second image.

2. The method of claim 1, wherein a screw entry point and a screw target point are set on the first screw path, and two normal lines each extending from the entry point or the target point in the first image pass through the second pedicle region of the second image.

3. The method of claim 1, wherein the first pedicle region has an inner edge facing the first vertebral body region and an outer edge at an opposite side to the inner edge, the entry point is located on or adjacent to the outer edge of the first pedicle region, and the target point is located within the first vertebral body region.

4. The method of claim 3, wherein the entry point and the target point in the second vertebral body region are set on an edge of the second pedicle region located within the second vertebral body region or set in the second pedicle region to be adjacent to the edge of the second pedicle region.

5. The method of claim 3, wherein the entry point is arranged on an edge of the second pedicle region toward an outer side of the second vertebral body region or arranged in the second pedicle region to be adjacent to the edge of the second pedicle region, and the target point is arranged on another edge facing the edge of the second pedicle region or arranged in the second pedicle region to be adjacent to the other edge.

6. The method of any one of claims 1 to 5, further comprising in the acquiring of the first image in the first direction and the second image in the second direction different from the first direction, calculating an angle of the second direction with respect to the first direction by detecting a posture of an image acquiring portion by using an optical tracking system (OTS).

7. The method of claim 6, wherein, in the registering, the first image and the second image are registered to the surgical space by reflecting the angle.

8. A two-dimensional (2D) image-based surgical planning system comprising:

an image acquisition device configured to acquire a first image in a first direction of a spinal surgery site and a second image in a second direction different from the first direction;
a spatial registration portion configured to define a virtual three-dimensional (3D) surgical space corresponding to the surgical site and register the first image and the second image to the surgical space;
an object separation portion configured to extract a first vertebral body region and a first pedicle region corresponding to a vertebra and a pedicle from the first image and extract a second vertebral body region and a second pedicle region within the second vertebral body region from the second image;
a path generating portion configured to set a first screw path passing through a center or a vicinity of the center of the first pedicle region in the first image, the first screw path being perpendicular to an intermediate boundary line passing between the first pedicle region and the first vertebral body region; and
a coordinate determining portion configured to determine a 3D coordinate for insertion of a screw in the surgical space by setting, based on the first screw path, an entry point and a target point of the screw corresponding to the first screw path in the second pedicle region of the second image.

9. The system of claim 8, wherein the path generating portion is further configured to set a screw entry point and a screw target point on the first screw path and allow two normal lines each extending from the entry point or the target point in the first image to pass through the second pedicle region of the second image.

10. The system of claim 8, wherein the first pedicle region has an inner edge facing the first vertebral body region and an outer edge at an opposite side to the inner edge, and
the path generating portion is further configured to allow the entry point to be located on the outer edge of the first pedicle region or in the first pedicle region to be adjacent to the outer edge and allow the target point to be located within the first vertebral body region.

11. The system of claim 10, wherein the path generating portion is further configured to set the entry point and the target point in the second vertebral body region on an edge of the second pedicle region located within the second vertebral body region or in the second pedicle region to be adjacent to the edge of the second pedicle region.

12. The system of any one of claims 8 to 11, wherein the coordinate determining portion is further configured to arrange the entry point on an edge of the second pedicle region toward an outer side of the second vertebral body region or in the second pedicle region to be adjacent to the edge and arrange the target point on another edge facing the edge of the second pedicle region or in the second pedicle region to be adjacent to the other edge.

13. The system of claim 12, further comprising an optical tracking system (OTS) configured to calculate an angle of the second direction with respect to the first direction by detecting a posture of the image acquisition device in a process of acquiring the first image in the first direction and the second image in the second direction different from the first direction.

14. The system of claim 13, wherein the registration portion is further configured to register the first image and the second image to the surgical space by reflecting the angle.

15. The system of any one of claims 8 to 11, further comprising an optical tracking system (OTS) configured to calculate an angle of the second direction with respect to the first direction by detecting a posture of the image acquisition device in a process of acquiring the first image in the first direction and the second image in the second direction different from the first direction.

16. The system of claim 15, wherein the registration portion is further configured to register the first image and the second image to the surgical space by reflecting the angle.

**Statement under Art. 19.1 PCT**

[0001] With respect to the violation of PCT Rule 6.1(a) noted in the Written Opinion, we have corrected the recitation of claim 16 from -13- to "15." With this correction, claims 14 and 16 do not claim the same invention, and thus the violation of PCT Rule 6.1(a) is resolved.

# FIG. 1

START

TWO-DIMENSIONAL (2D) X-RAY IMAGING (S1)

SPATIAL REGISTRATION AND
THREE-DIMENSIONAL (3D)
COORDINATE DEFINITION (S2)

AP/LL SEGMENTATION (S3)

TARGET LABELING (S4)

TARGET INPUT (S5)

PREPARATORY
TASK

LATERAL-LATERAL (LL)
PARAMETER EXTRACTION (S6)

LL OPTIMAL PATH POINT GENERATION (S7)

LL POINT
GENERATION

ANTERIOR-POSTERIOR (AP)
PARAMETER EXTRACTION (S8)

AP OPTIMAL PATH POINT GENERATION (S9)

AP POINT
GENERATION

OPTIMAL PATH GENERATION (S10)

(No)   LAST TARGET? (S11)

(Yes)

END

OPTIMIZATION
AND
AUTOMATION

# FIG. 2

# FIG. 3

(Input)

LL Segmentation model

(Output)

(Pedicle)

(Vertebra body)

# FIG. 4

(Input)
(Input)

AP
Segmentation
model

Pedicle

Vertebra
body

FIG. 5

FIG. 6

(Pedicle)

(Vertebra body)

# FIG. 7

(Pedicle)

(Vertebra body)

# FIG. 8

(AP)

Target(L1)
Target(L2)
Target(L3)
Target(L4)
Target(L5)

(LL)

Target(L1)
Target(L2)
Target(L3)
Target(L4)
Target(L5)

FIG. 9

(LL – L1)

(AP – L1)

FIG. 10

FIG. 11

FIG. 12

FIG. 13

FIG. 14

FIG. 15

FIG. 16

# FIG. 17

## INTERNATIONAL SEARCH REPORT

| International application No. |
| --- |
| **PCT/KR2023/017559** |

### A. CLASSIFICATION OF SUBJECT MATTER

**A61B 34/10**(2016.01)i; **G06T 7/30**(2017.01)i

According to International Patent Classification (IPC) or to both national classification and IPC

### B. FIELDS SEARCHED

Minimum documentation searched (classification system followed by classification symbols)

A61B 34/10(2016.01); A61B 17/56(2006.01); A61B 17/70(2006.01); A61B 6/00(2006.01); A61B 6/03(2006.01); A61B 6/12(2006.01); A61B 90/00(2016.01); G16H 30/40(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 척추 수술(backbone operation), 척추체(vertebra body), 척추경 (pedicle), 스크류 패스(screw path), 정합(registration)

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2394901 B1 (CUREXO INC.) 09 May 2022 (2022-05-09)<br>See paragraphs [0035]-[0053]. | 1-16 |
| A | KR 10-1264198 B1 (POSTECH RESEARCH AND BUSINESS DEVELOPMENT FOUNDATION) 14 May 2013 (2013-05-14)<br>See paragraphs [0035]-[0084]. | 1-16 |
| A | KR 10-2166149 B1 (CUREXO INC.) 15 October 2020 (2020-10-15)<br>See paragraph [0043]. | 1-16 |
| A | KR 10-2021-0157684 A (ELECTRONICS AND TELECOMMUNICATIONS RESEARCH INSTITUTE) 29 December 2021 (2021-12-29)<br>See paragraphs [0037]-[0119]. | 1-16 |

| ✓ Further documents are listed in the continuation of Box C. | ✓ See patent family annex. |
| --- | --- |

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **03 June 2024** | **03 June 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/017559** |

| C. | DOCUMENTS CONSIDERED TO BE RELEVANT | |
| --- | --- | --- |
| Category* | Citation of document, with indication, where appropriate, of the relevant passages | Relevant to claim No. |
| A | WO 2022-187659 A1 (NEW YORK SOCIETY FOR THE RELIEF OF THE RUPTURED AND CRIPPLED, MAINTAINING THE HOSPITAL FOR SPECIAL SURGERY) 09 September 2022 (2022-09-09)<br>    See claims 1-20. | 1-16 |

Form PCT/ISA/210 (second sheet) (July 2022)

## INTERNATIONAL SEARCH REPORT
### Information on patent family members

International application No.

**PCT/KR2023/017559**

| Patent document cited in search report | | | Publication date (day/month/year) | Patent family member(s) | | | Publication date (day/month/year) |
|---|---|---|---|---|---|---|---|
| KR | 10-2394901 | B1 | 09 May 2022 | CN | 115426967 | A | 02 December 2022 |
| | | | | EP | 4134034 | A1 | 15 February 2023 |
| | | | | JP | 2023-520602 | A | 17 May 2023 |
| | | | | KR | 10-2021-0123912 | A | 14 October 2021 |
| | | | | US | 11666458 | B2 | 06 June 2023 |
| | | | | US | 2023-0127917 | A1 | 27 April 2023 |
| | | | | WO | 2021-206372 | A1 | 14 October 2021 |
| KR | 10-1264198 | B1 | 14 May 2013 | None | | | |
| KR | 10-2166149 | B1 | 15 October 2020 | CN | 113543714 | A | 22 October 2021 |
| | | | | EP | 3939511 | A2 | 19 January 2022 |
| | | | | JP | 2022-524607 | A | 09 May 2022 |
| | | | | JP | 2023-116743 | A | 22 August 2023 |
| | | | | JP | 7355407 | B2 | 03 October 2023 |
| | | | | KR | 10-2020-0109641 | A | 23 September 2020 |
| | | | | US | 11666390 | B2 | 06 June 2023 |
| | | | | US | 2022-0039875 | A1 | 10 February 2022 |
| | | | | WO | 2020-185049 | A2 | 17 September 2020 |
| | | | | WO | 2020-185049 | A3 | 05 November 2020 |
| KR | 10-2021-0157684 | A | 29 December 2021 | KR | 10-2610915 | B1 | 06 December 2023 |
| | | | | US | 11928815 | B2 | 12 March 2024 |
| | | | | US | 2021-0398279 | A1 | 23 December 2021 |
| WO | 2022-187659 | A1 | 09 September 2022 | AU | 2022-231159 | A1 | 09 September 2022 |
| | | | | CA | 3209143 | A1 | 09 September 2022 |
| | | | | CN | 117241757 | A | 15 December 2023 |
| | | | | EP | 4301268 | A1 | 10 January 2024 |
| | | | | IL | 305327 | A | 01 October 2023 |
| | | | | JP | 2024-510140 | A | 06 March 2024 |
| | | | | KR | 10-2024-0016247 | A | 06 February 2024 |
| | | | | WO | 2022-187659 | A9 | 12 October 2023 |

Form PCT/ISA/210 (patent family annex) (July 2022)

**EP 4 775 164 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2203544